# EUROPEAN PATENT APPLICATION

(11) **EP 1 775 347 A2**
(43) Date of publication of application: **18.04.2007**
(21) Application number: 06021182.8
(22) Date of filing: 09.10.2006
(51) Int. Cl.: C12P 17/10, C12P 41/00

(54) **A process for the enantiomeric resolution of 1-substituted 2-(aminomethyl)-pyrrolidines by amidation with lipases**

(30) Priority: 14.10.2005 IT MI20051943
(71) Applicant: Procos S.p.A., 28062 Cameri NO (IT)
(72) Inventor: Bertolini, Giorgio, 20099 Sesto San Giovanni (MI) (IT); Bogogna, Luigi, 28062 Cameri (NO) (IT); Pregnolato, Massimo, 27020 Carbonara al Ticino (PV) (IT); Terreni, Marco, 20154 Milano (IT); Velardi, Francesco, 28062 Cameri (NO) (IT)
(74) Representative: Minoja, Fabrizio

(57) **Abstract**

A process for the enantiomeric resolution of 1-substituted 2-(aminomethyl)pyrrolidines of formula (I) 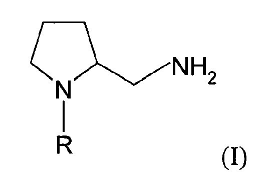 in which R is C1-C6 alkyl,
which process comprises the reaction of the racemic amine with benzyl acetate in acetonitrile in the presence of a lipase selected from *Pseudomonas cepacia, Pseudomonas fluorescens* or *Candida rugosa* lipases, to give the corresponding 1-substituted N-(pyrrolidin-2-yl-methyl)-acetamides of formula (II), with R configuration, 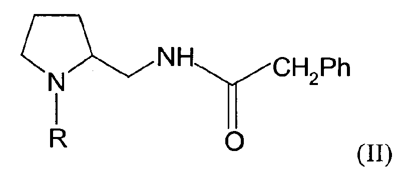 and the residual amine with S configuration,
and, if desired, the subsequent hydrolysis of the amide to obtain the amine with R configuration.

## Description

The present invention relates to a process for the enantiomeric resolution of 1-substituted 2-(aminomethyl)pyrrolidines by amidation in the presence of lipases.

Optically active 1-substituted 2-(aminomethyl)pyrrolidines are useful intermediates for the preparation of pharmaceutical active ingredients, in particular for the preparation of Levosulpiride and enantiomerically pure forms of similar medicaments, such as Sultopride and Amilsulpiride.

Levosulpiride, of formula (II) reported below, specifically requires 2-(aminomethyl)-1-ethyl-pyrrolidine of S configuration as intermediate.

(S) 2-(Aminomethyl)-1-ethyl pyrrolidine can be prepared by chemical conversion of proline through multi-step reactions which make use of expensive, dangerous reactants.

Conventional optical resolution methods have drawbacks connected with the use of expensive optically active reactants, unsatisfactory yields and cumbersome, seldom efficient procedures for the racemization of the undesired R isomer.

A particularly efficient process has now been found for the enantiomeric resolution of 1-substituted 2-(aminomethyl)pyrrolidines of formula I 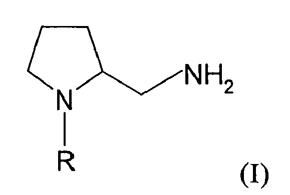 in which R is C1-C6 alkyl.

The process of the invention, characterized by the use of microbial lipases, provides the desired enantiomer of S configuration with enantioselectivity higher than 95%, preferably higher than 99%.

The process of the invention comprises the reaction of the racemic amine with benzyl acetate in acetonitrile in the presence of a lipase selected from *Pseudomonas cepacia, Pseudomonas fluorescens* or *Candida rugosa* lipases, to give the corresponding 1-substituted N-(pyrrolidin-2-yl-methyl)-acetamides of formula (II), with R configuration, 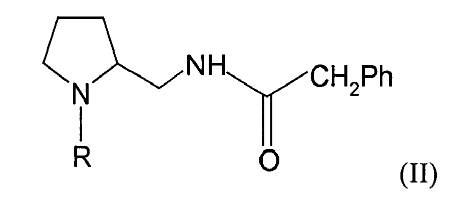 and the residual amine with (S) configuration.

Following the enzymatic reaction, the (R) amide of formula (II) is subjected to hydrolysis to afford the (R) amine.

A number of tests carried out with different acylating agents, enzymes and solvents, surprisingly proved that only the combination of the enzymes from *Pseudomonas cepacia, Pseudomonas fluorescens* or *Candida rugosa* with benzyl acetate as the acylating agent and acetonitrile as the solvent not only provides the desired optical resolution but also affords substantially quantitative yields. In fact, when using a different solvent or a different acylating agent, either yields are unsatisfactory or there is no resolution at all. For example, acylating agents such as trifluoroethyl butyrate, ethyl butyrate, allyl butyrate, diallyl carbonate, methyl mandelate, methylphenyl acetate and solvents such as isopropyl ether, tert-butyl methyl ether, octane, dioxane, always give unsatisfactory results.

The process according to the invention is preferably carried out at room temperature, for times ranging from 48 to 172 hours.

Enzymes from *Pseudomonas cepacia, Pseudomonas fluorescens* or *Candida rugosa* are commercially available and, as a rule, they are used in amounts ranging from 300 to 1000 units per g of substrate. If desired, said enzymes can be bound to suitable supports, according to conventional techniques.

Furthermore, the invention relates to a process for the preparation of Levosulpiride, which comprises the resolution of 2-(aminomethyl)-1-ethyl pyrrolidine by reacting the racemic amine with benzyl acetate in acetonitrile in the presence of a lipase selected from *Pseudomonas cepacia, Pseudomonas fluorescens* or *Candida rugosa* lipases, to give the corresponding N-(pyrrolidin-2-yl-methyl)-1-ethyl acetamide, with R configuration, and the residual amine with S configuration which is isolated and purified by distillation. The pure amine with S configuration is then converted to Levosulpiride by reaction with a 2-methoxy-5-sulfamoylbenzoic acid derivative such as methyl 2-methoxy-5-sulfamoylbenzoate in alcohols such as methanol, ethanol, propanol or butanol at a temperature ranging from 20°C to the reflux temperature of the solvent, and subsequent purification according to known techniques such as extractions and/or crystallizations.

The following examples illustrate the invention in greater detail.

### EXAMPLE 1

A mixture of 100 mmoles of R,S-2-(aminomethyl)-1-ethyl-pyrrolidine (AMEP), 200 mmoles of benzyl acetate, in 1 liter of acetonitrile, was added with an amount of a crude extract of a lipase from *Pseudomonas fluorescens* (PFL), *Pseudomonas cepacia* (PCL) or *Candida rugosa* (CRL), equivalent to 25 mg/ml. The mixture was kept at room temperature (20-25°C) for the times indicated below.

For monitoring the reactions, samples were taken at different times, then centrifuged to remove the enzyme, and the conversion degree of the amine to amide was evaluated by GC analysis of the supernatant from centrifugation.

The supernatant was directly analyzed injecting 1 µl of solution on a SPB-5 column (50 m length, 0.32 mm i.d., polymethylsiloxane film thickness 0.45 µm) using helium as the carrier and a flame ionization detector, according to the temperature program reported in Table 1.

**Table 1**

| **Tim (min)** | **Temperature (°C)** | **Rate (°C/min)** | **Comments** |
|---|---|---|---|
| 0-5 | 50 | - | Isotherm |
| 5-16.5 | 50→280 | 20 | linear gradient |
| 16.5-37 | 280 | - | Isotherm |

The conversion degree was calculated using the values of the areas of the two peaks of the starting product and the acetylated product, respectively.

After reaching the desired conversion degree, the reaction mixture was analyzed by HPLC analysis, to evaluate the optical purity of the residual amine, according to the procedure reported in the following:

A reaction sample was evaporated to dryness under nitrogen, then the residue was redissolved in ethanol to a final concentration of about 100 mg/ml. 20 µl of this solution was injected on a CHIRALPAK ADH column 250 x 4.6 mm using an n-hexane-absolute ethanol-diethylamine 80:20:0.75 mixture as a mobile phase with flow of 1.0 ml/min and UV detector set at 220 nm.

Results were expressed as peaks area percentages, only considering the peaks of the two enantiomers.

The results are reported in Table 2 below.

**Table 2**

| | **Time (hours)** | **% Synthesis *** | **%S*** | **%R*** | **% eeₛ** |
|---|---|---|---|---|---|
| PCL | 168 h | 61% | 100 | 0 | > 99 (S) |
| PFL | 48 h | 63% | 100 | 0 | > 99 (S) |
| CRL | 168 h | 43% | 100 | 0 | > 99 (S) |

After reaching the desired conversion degree, the reaction mixture was evaporated to dryness and (S)-2-(aminomethyl)-1-ethylpyrrolidine was isolated and purified by distillation under vacuum, collecting the fractions between 40 and 45°C at 10 mmHg.

### EXAMPLE 2

A mixture of (S)-2-(aminomethyl)-1-ethylpyrrolidine (143 g) and methyl 2-methoxy-5-sulfamoylbenzoate (260 g) in n-butanol (1040 ml) was refluxed for 20 hours, then cooled to room temperature and extracted with a solution of concentrated hydrochloric acid (115 g) in water (1040 ml). The aqueous phase was then alkalinized with concentrated ammonia (about 95 g) and the resulting product was filtered and dried, to obtain 277 g of Levosulpiride (75% molar yield) that, if desired, can be recrystallized from alcohols such as methanol or ethanol.

## Claims

1. A process for the enantiomeric resolution of 1-substituted 2-(aminomethyl)pyrrolidines of formula (I) 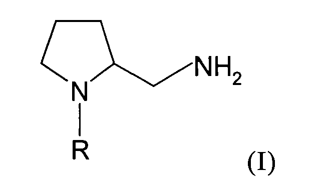 in which R is C1-C6 alkyl,
which process comprises the reaction of the racemic amine with benzyl acetate in acetonitrile in the presence of a lipase selected from *Pseudomonas cepacia, Pseudomonas fluorescens* or *Candida rugosa* lipases, to give the corresponding 1-substituted N-(pyrrolidin-2-yl-methyl)-acetamides of formula (II), with R configuration, 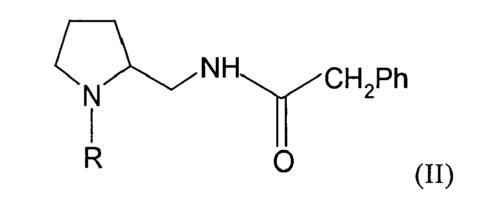 and the residual amine with S configuration,
and, if desired, the subsequent hydrolysis of the amide to obtain the amine with R configuration.

2. A process as claimed in claim 1, which is carried out at room temperature.

3. A process as claimed in claim 1 or 2, in which enantioselectivity for the (S) isomer is higher than 95%.

4. A process as claimed in claim 3, in which enantioselectivity for the isomer of S configuration is higher than 99%.

5. A process as claimed in any one of claims 1 to 4, in which the reaction is carried out for times ranging from 24 to 172 hours.

6. A process for the preparation of Levosulpiride, which comprises the resolution of 2-(aminomethyl)-1-ethyl pyrrolidine by reaction of the racemic amine with benzyl acetate in acetonitrile in the presence of a lipase selected from *Pseudomonas cepacia, Pseudomonas fluorescens* or *Candida rugosa* lipases, to give the corresponding N-(pyrrolidin-2-yl-methyl)-1-ethyl acetamide and the residual (S) amine, the subsequent reaction of the resulting optically active (S) amine with methyl 2-methoxy-5-sulfamoylbenzoate in alcoholic solvent, such as methanol, ethanol, propanol or butanol, at a temperature ranging from 20°C to the solvent's reflux temperature, and the subsequent purification according to conventional known techniques such as extractions and/or crystallizations.
